(19) Europäisches Patentamt
European Patent Office
Office européen des brevets

(11) Veröffentlichungsnummer: **0 305 908 B1**

(12) **EUROPÄISCHE PATENTSCHRIFT**

(45) Veröffentlichungstag der Patentschrift: **18.03.92**

(51) Int. Cl.5: **C07D 301/26**, C07D 303/02

(21) Anmeldenummer: **88113906.7**

(22) Anmeldetag: **26.08.88**

(54) **Verfahren zur Herstellung von phenyl-substituierten Epoxiden.**

(30) Priorität: **02.09.87 DE 3729226**

(43) Veröffentlichungstag der Anmeldung:
**08.03.89 Patentblatt 89/10**

(45) Bekanntmachung des Hinweises auf die Patenterteilung:
**18.03.92 Patentblatt 92/12**

(84) Benannte Vertragsstaaten:
**AT BE CH DE ES FR GB GR IT LI NL SE**

(56) Entgegenhaltungen:
**CH-A- 433 264**
**US-A- 2 887 509**

(73) Patentinhaber: **BASF Aktiengesellschaft**
**Carl-Bosch-Strasse 38**
**W-6700 Ludwigshafen(DE)**

(72) Erfinder: **Goetz, Norbert, Dr.**
**Schoefferstrasse 25**
**W-6520 Worms 1(DE)**
Erfinder: **Recker, Hans-Gert, Dr.**
**Lisztstrasse 117**
**W-6700 Ludwigshafen(DE)**
Erfinder: **Smuda, Hubert, Dr.**
**Rahmengasse 32**
**W-6900 Heidelberg(DE)**

EP 0 305 908 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung phenylsubstituierter Epoxide.

Phenylsubstituierte Epoxide sind wertvolle Zwischenprodukte zur Herstellung antimykotisch und fungizid wirksamer Azolylmethyloxirane. Derartige Azolylmethyloxirane und deren Herstellung werden in der europäischen Patentanmeldung 94 564 beschrieben.

Die technische Herstellung von phenylsubstituierten Epoxiden wie z. B. Styroloxid erfolgt z.B. durch Friedel-Crafts-Alkylierung von Benzol mit Ethylen zum Ethylbenzol und anschließender Dehydrierung zum Styrol. Die Epoxidierung kann mittels nachfolgender Gasphasenoxidation, mittels Persäure oder Anlagerung von Hypochloriger Säure mit anschließendem Ringschluß erfolgen (vgl. z. B. K. Weissermel, H. J. Arpe: Industrielle organische Chemie, 2. Auflage, S. 256 und 318 - 321, Verlag Chemie, Weinheim sowie Org. Synth. Coll. Vol. I, 494 (1948)).

Will man gezielt substituierte Phenylepoxide erhalten, so wird man den Weg über eine Friedel-Crafts-Alkylierung vermeiden, denn diese Reaktion führt in der Regel zu Produktgemischen. Diese Gemische sind meist schwierig zu trennen und daher für die technische Herstellung dieser Verbindungen nicht brauchbar.

Bessere Ausgangsstoffe stellen daher die mit guter Selektivität synthetisierbaren substituierten Benzaldehyde sowie substituierten omega-Halogenacetophenone dar.

Ausgehend von Benzaldehyden läßt sich das zugehörige Epoxid z.B. mittels Umsetzung mit dem Anion eines Trimethylsulfoniumhalogenids erzeugen. (vgl. z. B. V. Franzen, H.E. Driesen, Chem. Ber. 96, 1881 (1963)). Jedoch sind die Benzaldehyde oftmals nur in mäßiger Ausbeute zugänglich (vgl. z. B. Oláah et al., Acta Chim. Hung. 7, 89 (1955); Lock et al., Chem. Ber. 72, 1064, (1939); Brown et al., J. Chem. Soc. 1949, Suppl. 95, 99; Hass et al. J.A.C.S. 71, 1767, (1949)).

Ausgehend von substituierten omega-Halogenacetophenonen, die in guter bis sehr guter Ausbeute erhalten werden können, sind die zugehörigen Epoxide durch Reduktion der Carbonylgruppe zum Halogenhydrin mittels geeigneter Reagentien wie Aluminium-tri-isopropanolat nach Meerwein, Ponndorf und Verley (vgl. Organikum, 11. Aufl. 1972, S. 539 sowie C.0. Guss, J. Org. Chem. 17, 678 (1952)) oder Alkali- bzw. Erdalkaliborhydrid zugänglich. (vgl.
A.C. Knipe, J. Chem. Soc. Perkin Trans. II, 589 (1973)
A.P. Bossetti, D.R. Crist, J. Het. Chem. 12, 1287 (1975)
H. Hopff, R. Wandeler, Helv. Chim. Acta, Vol. XLV, 982 (1962)
M. Sato, A. Kosasayama, F. Uchimaru,
Chem. Pharm, Bull. 29, 2885 (1981)
C. Guss, J. Org. Chem. 17, 678 (1952)
J.F. Nash US Pat. 2 887 509
W. Wierenga, A.W. Harrison, B.R. Evans, C.G. Chidester,
J. Org. Chem. 49, 438 (1984)
L. Almiranze, W. Murmann Öster. Patent 25 22 15
Continental Pharma, Belg. Pat. 877 694
Hoffmann-La Roche, Fr. Pat. 1 381 639
N.B. Chapman, K. Clarke, R.M. Pinder, S.N. Shawney
J. Chem. Soc. (C) 293, (1967)
S. Sorriso, Zeitschr. f. Naturf. B 32, 1467 (1977)
A. Huth, F. Neubauer, Liebigs Ann. Chem. 56 (1979)
Ciba-Geigy, Europ. Pat. 69 838
Kiernan, J.A., Baker, P.K., EP 0026 298
Abenheim, Henry-Basch, Freon, Bull. Soc. Chim. France 179 (1970)).

Nachfolgender Ringschluß durch Alkali- oder Erdalkalihydroxide führt zu dem gewünschten Epoxid (siehe Formelschema)

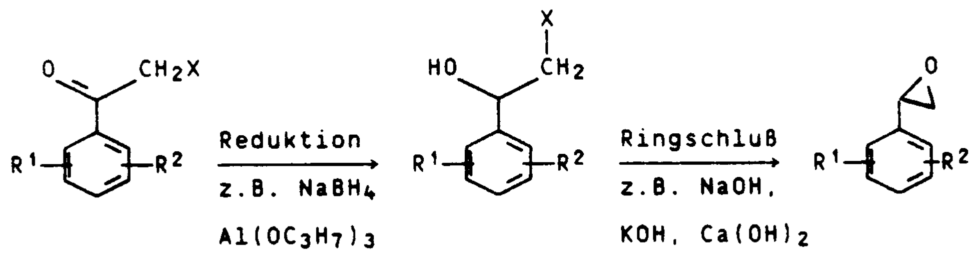

Da bekannt ist, daß omega-Halogenketone unter dem Einfluß von Hydroxyl-Ionen in protischen Lösungsmitteln viele Nebenreaktionen eingehen, wie z. B. Hydrolyse des omega-Halogenatoms (vgl. R. Verhé, N. DeKimpe in S. Patai (Hrsg.): The Chemistry of Functional Groups, Suppl. D, Part 1: Halides, Pseudo-Halides and Azides, J. Wiley & Sons, 1983, S. 850 ff und dort zitierte Literatur), werden in der Fachliteratur die beiden Reaktionsschritte, Reduktion und Ringschluß, bei der Arbeitsweise in protischen Lösungsmitteln in dieser Reihenfolge nacheinander und voneinander getrennt ausgeführt beschrieben (vgl.
A.C. Knipe, J. Chem. Soc. Perkin Trans. II, 589 (1973)
A.P. Bossetti, D.R. Crist, J. Het. Chem. 12, 1287 (1975)
H. Hopff, R. Wandeler. Helv. Chim. Acta, Vol. XLV, 982 (1962)
M. Sato, A. Kosasayama, F. Uchimaru,
Chem. Pharm, Bull. 29. 2885 (1981)
C. Guss, J. Org. Chem. 17, 678 (1952)
J.F. Nash US Pat. 2 887 509
W. Wierenga, A.W. Harrison, B.R. Evans, C.G. Chidester,
J. Org. Chem. 49. 438 (1984)
L. Almiranze, W. Murmann Öster. Patent 25 22 15
Continental Pharma, Belg. Pat. 877 694
Hoffmann-La Roche. Fr. Pat. 1 381 639
N.B. Chapman, K. Clarke, R.M. Pinder, S.N. Shawney
J. Chem. Soc. (C) 293, (1967)
S. Sorriso, Zeitschr. f. Naturf. B 32, 1467 (1977)
A. Huth, F. Neubauer, Liebigs Ann. Chem. 56 (1979)
Ciba-Geigy, Europ. Pat. 69 838
Kiernan. J.A., Baker, P.K., EP 0026 298
Abenheim, Henry-Basch, Freon Bull. Soc. Chim. France 179 (1970)).

Dies gilt sowohl für die Reduktion nach Meerwein, Ponndorf und Verley als auch für die Reduktion mit Natriumborhydrid. Zum Einsatz kommen verdünnte Reagenslösungen wie z. B. 2N $NaBH_4$-Lösung sowie 2N KOH-Lösung (vgl. z. B. N.B. Chapman. K. Clarke, R.M. Pinder, S.M. Sawhney. J. Chem. Soc. C. 1967, 293). Für die Arbeitsweise mit Natriumborhydrid in protischen Lösungsmitteln werden in der Fachliteratur folgende stöchiometrischen Verhältnisse empfohlen (berechnet auf Reduktionsäquivalente des Borhydrids):

| mol-Verhältnis omega-Halogenacetophenon/ Natriumborhydrid | Überschuß an Reduktionsäquivalenten | Literatur |
|---|---|---|
| 1,72 : 2,43 | 5,6-fach | US-2.887.509 |
| 1,63 : 0,78 | 2-fach | AU-25 22 15 |
| 0,1 : 0,11 | 4-fach | BE-877 694 |
| 0,04 : 0,05 | 4-fach | N.B. Chapman et al J. Chem. Soc. (C) 293 (1967) |
| 0,096 : 0,063 | 2,6-fach | S. Sorriso. Zeitschr. f. Naturf. B 32, 1467 (1977) |
| 0,11 : 0,21 | 7,6-fach | E.P. 26 298 |

Demnach muß der Fachmann davon ausgehen, daß unter den angewendeten Reaktionsbedingungen Zersetzungsreaktionen des Reduktionsmittels ablaufen.

Die bekannten Verfahren sind insofern nachteilig, als dort ein großer Überschuß an Reduktionsmittel angewendet wird, ein zusätzlicher Verfahrensschritt durch extraktive Trennung (Abtrennung des Halogenhydrins von der Reduktionsmischung) erforderlich ist, das Lösungsmittel gewechselt werden muß, oder aber nicht näher beschriebene Mischungen von Epoxiden und Halogenhydrin erhalten werden (vgl. J.F. Nash, US Patent 2 887 509 und C.0. Guss, J.Org. Chem. 17, 678 (1952)).

Der vorliegenden Erfindung liegt die Aufgabe zugrunde, ein einfaches und ökonomisches Verfahren zur Herstellung phenylsubstituierter Epoxide in hohen Ausbeuten zur Verfügung zu stellen.

Diese Aufgabe wird gelöst durch Bereitstellung eines Verfahrens zur Herstellung Phenyl-substituierter Epoxide der Formel I

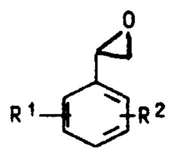

I,

worin $R^1$ und $R^2$ gleich oder verschieden sind und für Wasserstoff, $C_1$-$C_4$-Alkyl, $C_1$-$C_4$-Alkenyl, Phenyl, Halogen, Nitro, $C_1$-$C_4$-Alkoxy, $C_1$-$C_4$-Halogenalkyl, Phenoxy oder Phenylsulfonyl stehen, durch Reduktion eines Halogenacetophenons der Formel II

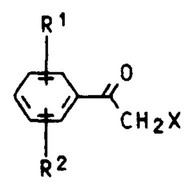

II,

worin $R^1$ und $R^2$ die obengenannten Bedeutungen besitzen und X für ein Halogenatom steht, mit einem Alkaliborhydrid zu einem Halogenhydrin der Formel III

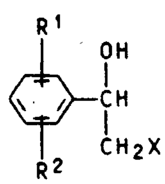

III,

worin $R^1$, $R^2$ und X die oben genannten Bedeutungen haben, und Umsetzung des Halogenhydrins mit einem Alkali- oder Erdalkalihydroxid, indem man die Lösung beziehungsweise Suspension aus Alkaliborhydrid und Alkali- oder Erdalkalihydroxid in einem Gemisch aus Wasser und einer mit Wasser mischbaren organischen Flüssigkeit mit einer Lösung des Halogenacetophenons in einer organischen Flüssigkeit umsetzt.

Man verwendet das Halogenacetophenon z.B. in Form seiner Lösung in einer mit Wasser mischbaren oder mit Wasser nicht mischbaren organischen Flüssigkeit und setzt es mit einer konzentrierten Lösung oder Suspension eines Alkali- oder Erdalkalihydroxid in einem Gemisch aus Wasser und einer mit Wasser mischbaren organischen Flüssigkeit um. Hierfür geeignete organische Flüssigkeiten sind mit Wasser mischbare Alkohole, Amide, Nitrile, Sulfoxide, Sulfone, Harnstoffe und Ether.

Bevorzugt wird eine Lösung einer Mischung aus Alkaliborhydrid und Alkalihyroxid in einem Gemisch aus Wasser und einer mit Wasser mischbaren organischen Flüssigkeit mit einer Lösung des Halogenacetophenons in einer organischen Flüssigkeit umgesetzt.

Es wurde überraschend festgestellt, daß die bekannten Nebenreaktionen der omega-Halogenacetophenone bei dieser Verfahrensweise nicht beobachtet werden und die phenylsubstituierten Epoxide in guter bis

sehr guter Ausbeute erhalten werden. Nach dem erfindungsgemäßen Verfahren lassen sich insbesondere Verbindungen der Formel I herstellen bei denen $R^1$ und $R^2$ nicht gleichzeitig Wasserstoff bedeuten.

Das erfindungsgemäße Verfahren nach Anspruch 1 kann kontinuierlich oder diskontinuierlich z.B. in einem Rührkessel durchgeführt werden. Die Umsetzung erfolgt zweckmäßig z.B. in einem Temperaturbereich von +10 °C bis +60 °C, bevorzugt bei +10 °C bis +40 °C, besonders bevorzugt bei +20 °C bis +40 °C.

Die Abtrennung des Endproduktes aus der Reaktionsmischung kann durch übliche Methoden der präparativen organischen Chemie erfolgen, z. B. durch Extraktion und anschließende Destillation oder Umkristallisation mit einem organischen Lösungsmittel. Zur Extraktion kann der pH-Wert der wässrigen Phase durch Zusatz von Säure, z.B. 10 - 100%iger Säure, vorzugsweise 20 -50%iger Säure auf pH 2 bis pH 14 eingestellt werden. Es wurde überraschend festgestellt, daß die Ausbeute bei der Extraktion abhängig von dem pH-Wert der wässrigen Phase ist, so daß besonders bevorzugt ein pH-Wert von pH 4 - 9 oder 12 - 14 eingestellt wird.

Das omega-Halogenacetophenon der Formel II kann in Form einer Lösung in einem mit Wasser mischbaren oder mit Wasser nicht mischbaren organischen Lösungsmittel eingesetzt werden. Die Konzentrationen der Lösungen können z.B. von 5 Gew.-% bis zur Sättigungskonzentration an Halogenacetophenon betragen, bevorzugt werden die gesättigten Lösungen verwendet. Zu den bevorzugten mit Wasser mischbaren organischen Lösungsmitteln gehören Alkohole wie Methanol, Ethanol, Isopropanol, n-Propanol, n-Butanol, sec.-Butanol, tert.-Butanol, Ethylenglykol, Propylenglykol, Glyzerin, aber auch cyclische Amide wie N-Methylpyrrolidon, Nitrile wie Acetonitril, Sulfoxide bzw. Sulfone wie Dimethylsulfoxid oder Sulfolan, Harnstoffderivate wie 1,3-Dimethyl-3,4,5-tetrahydro-2(1H)pyrimidon (DMPU) oder Ether wie Tetrahydrofuran oder Glykolether oder Mischungen dieser Flüssigkeiten.

Zu den bevorzugten mit Wasser nicht mischbaren organischen Lösungsmitteln gehören Alkohole z. B. Benzylalkohol, Pentanol, Cyclohexanol, Cyclopentanol, halogenierte Kohlenwasserstoffe z. B. Methylenchlorid, Chloroform, Tetrachlorkohlenstoff, Dichlorethan, Chlorbenzol, Fluorbenzol, Kohlenwasserstoffe wie Ligroin, Toluol, Benzol, Xylol, Cyclohexan, Pentan, Hexan, Heptan oder Octan oder Ether z. B. Methyl-tert.-Butylether.

Geeignete Basen sind z. B. Alkalihydroxide, wie Lithium-, Natrium- oder Kaliumhydroxid, ggfs. unter Zusatz eines Phasentransferkatalysators (vgl. E.V. Dehmlow. S.S. Dehmlow, Phase Transfer Catalysis, Verlag Chemie 1980). Ebenso geeignet sind z.B. Erdalkalihydroxide wie Magnesiumhydroxid, Calcium-, Strontium- oder Bariumhydroxid. Besonders bevorzugt werden Natrium- und Kaliumhydroxid sowie Calciumhydroxid in Form ihrer wäßrigen Lösungen bzw. Suspensionen in einer Konzentration von z.B. 20 % bis 60 %, bevorzugt 28 % bis 50 % (Gew.-%) eingesetzt.

Geeignete Reduktionsmittel sind z.B. die Alkali- und Erdalkalisalze des Tetrahydridoborats, aber auch durch Alkoxy- oder Cyanogruppen substituiertes Tetrahydridoborat kann eingesetzt werden. Bevorzugt wird Natriumtetrahydridoborat (Natriumboranat, $NaBH_4$) eingesetzt, besonders bevorzugt in Form einer 6,6 bis 22,5 Gew.-% $NaBH_4$-enthaltenden Lösung in 28 bis 50 Gew.-% Na0H enthaltender wässriger Lösung.

Die Reduktionsmittel werden bevorzugt in stöchiometrischer Menge bezogen auf das omega-Halogen-Acetophenon der Formel II eingesetzt, können aber auch bis zum doppelten stöchiometrischen Überschuß angewendet werden.

Die Basen werden bevorzugt in stöchiometrischer Menge, bezogen auf das omega-Halogen-Acetophenon der Formel II, eingesetzt, können aber auch bis zum doppelten stöchiometrischen Überschuß angewendet werden.

Das einstufige Verfahren erlaubt eine technisch vorteilhafte Durchführung der Synthese phenylsubstituierter Epoxide der Formel I, da ein extraktiver Aufarbeitungsschritt des Halogenhydrins entfällt.

In den Verbindungen der Formel I stehen die Reste $R^1$ und $R^2$ die gleich oder verschieden voneinander sein können, vorzugsweise für Wasserstoff, Methyl, Ethyl, Propyl, Isopropyl, Butyl, sek.-Butyl, iso-Butyl, tert.-Butyl, Phenyl, 2-Chlor, 3-Chlor, 4-Chlor, 2-Fluor, 3-Fluor, 4-Fluor, 4-Brom, 2-Fluor-6-Chlor, 2,4-Dichlor, 3,4-Dichlor, 3,5-Dichlor, 2,6-Dichlor, 3-Chlor-4-methyl, 2-Methoxi, 3-Methoxi, 2,4-Dimethoxi, 3,4-Dimethoxi, 4-Methoxi, 4-Ethoxi, 4-tert.-Butoxi, 4-Phenoxi, 3-Phenoxi, 3-Nitro, 4-Nitro, 3-Trifluormethyl, 4-Trifluormethyl oder 4-Phenylsulfonyl.

Besonders bevorzugt bedeuten die Reste $R^1$ und $R^2$ Wasserstoff, 2-Chlor, 4-Chlor, 2-Fluor, 3-Fluor, 4-Fluor, 2-Fluor-6-Chlor, 4-Brom, 2,4-Dichlor oder Halogenmethyl, mit der Maßgabe, daß $R^1$ und $R^2$ nicht gleichzeitig für Wasserstoff stehen.

Die Phenyl-substituierten Epoxide können an Siliciumdioxid-Katalysatoren zu den entsprechenden Phenylacetaldehyden

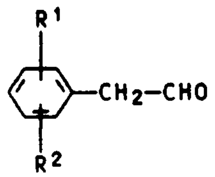

umgelagert werden, die mit Aldehyden zu den Acroleinen

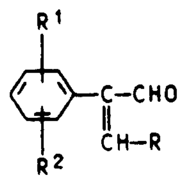

kondensiert werden können.

Diese können z.B. mit $H_2O_2$ zu den Formyloxiranen

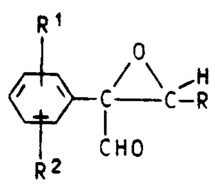

oxidiert werden, die durch Reduktion z.B. mit $NaBH_4$ zu den Hydroxymethyloxiranen

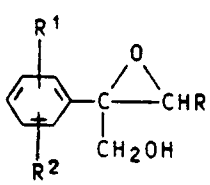

umgesetzt werden können, die für die in EP-94 564 beschriebene Umsetzung zu Azolylmethyloxiranen geeignet sind.

Herstellvorschriften

Beispiel 1

4-Fluorphenyloxiran (p-Fluorstyroloxid)

In einer Lösung von 2,1 mol Alkalihydroxid oder einer Aufschlämmung von 1,1 mol Erdalkalihydroxid in 200 ml Wasser werden unter $N_2$-Atmosphäre 0,52 mol Natriumboranat bei Raumtemperatur aufgelöst. Diese Lösung wird in einem Wasserbad gekühlt. Anschließend werden 10 - 800 ml eines Alkohols (s. Tabelle 1) so langsam zugegegeben, daß die Temperatur 35°C nicht übersteigt. Nach vollständiger Mischung der Komponenten wird auf 20°C abgekühlt und dann 345 g (2 mol) omega-Chlor-p-fluorphenylacetophenon in Form einer 20 bis 70 gew.-%igen Lösung, vorzugsweise einer gesättigten Lösung, in einem mit Wasser nicht mischbaren oder mit Wasser mischbaren organischen Lösungsmittel (s. Tabelle 1) so langsam zugegeben, daß die Innentemperatur der Reaktionsmischung 55°C nicht übersteigt. Nach beendeter Zugabe wird 2 min bis 6 Tage nachgerührt.

Aufarbeitung

A. Wenn wassermischbare organische Lösungsmittel verwendet werden:

200 - 400 ml eines mit Wasser nicht mischbaren organischen Lösungsmittels (z.B. Toluol) werden zugegeben. In der wäßrigen Phase wird mit 10 bis 100%iger Säure, vorzugsweise 50%iger Schwefelsäure ein pH-Wert von pH 4 bis pH 14, vorzugsweise pH 7, eingestellt. Nach der Phasentrennung von der wäßrigen Lösung wird die organische Phase destilliert. Die als Vorlauf übergehenden organischen Lösungsmittel können wieder verwendet werden. Das Endprodukt kann bei verschiedenen Drucken in derselben Apparatur oder mittels eines Dünnfilmverdampfers destilliert werden. Ausbeuten s. Tabelle 1.

B. Wenn mit Wasser nicht mischbare organische Lösungsmittel eingesetzt werden:

Dem Ansatz werden 100 - 400 ml Wasser zugegeben. Der pH-Wert der wäßrigen Phase wird mit Säure wie unter A. auf pH 4 bis pH 14, vorzugsweise pH 7, eingestellt. Die wäßrige Phase wird abgetrennt und die organische Phase destilliert. Die als Vorlauf übergehenden organischen Lösungsmittel können wieder verwendet werden. Das Endprodukt kann bei verschiedenen Drucken in derselben Apparatur oder mittels eines Dünnfilmverdampfers destilliert werden.

Tabelle 1: Ausbeuten an 4-Fluorphenyloxiran (Kp.$_{24}$ = 91 - 93$^0$C) entsprechend Beispiel 1:

| Ansatz | Menge und Art der Base | Menge $NaBH_4$ | Alkohol-komponente (m. Wasser mischbar) | Lösungsmittel f. Acetophenon (m.Wasser nicht mischbar außer 13) | Temp. | Reakt. dauer | Ausbeute (Gew.%) n. Destillation (bezogen auf Halogen-acetophenon) |
|---|---|---|---|---|---|---|---|
| 1 | 84 g NaOH | 19 g | 286 ml Isopropanol | 300 ml $CH_2Cl_2$ | RT (20$^0$C) | 4 h | 96 |
| 2 | 81 g $Ca(OH)_2$ | 19 g | 400 ml Methanol | 700 ml $CH_2Cl_2$ | RT | 14 h | 90 |
| 3 | 81 g $Ca(OH)_2$ | 19 g | 400 ml Methanol | 350 ml $CH_2Cl_2$ | RT | 14 h | 89 |
| 4 | 81 g $Ca(OH)_2$ | 19 g | 400 ml Methanol | 350 ml $CH_2Cl_2$ | RT | 12 h | 96 |
| 5 | 81 g $Ca(OH)_2$ | 19 g | 400 ml Methanol | 690 ml Toluol | RT | 14 h | 93 |
| 6 | 81 g $Ca(OH)_2$ | 19 g | 400 ml Methanol | 690 ml Toluol | RT | 14 h | 96 |
| 7 | 81 g $Ca(OH)_2$ | 19 g | 400 ml Methanol | 690 ml Toluol | Rückfluß (80$^0$C) | 1 h | 84 |

Tabelle 1: Fortsetzung- Ausbeuten an 4-Fluorphenyloxiran (Kp.$_{24}$ = 91 - 93$^0$C):

| Ansatz | Menge und Art der Base | Menge $NaBH_4$ | Alkohol-komponente (m. Wasser mischbar) | Losungsmittel f. Acetophenon (m. Wasser nicht mischbar außer 13) | Temp. | Reakt. dauer | Ausbeute (Gew.-%) n. Destillation (bezogen auf Halogen-acetophenon) |
|---|---|---|---|---|---|---|---|
| 8 | 84 g NaOH | 19 g | 300 ml Methanol | 172 ml $CH_2Cl_2$ | RT | 1 h | 96 |
| 9 | 84 g NaOH | 19 g | 40 ml Methanol | 172 ml $CH_2Cl_2$ | RT | 15 min | 86 |
| 10 | 118 g KOH | 19 g | 150 ml Methanol | 300 ml Toluol | RT | 30 min | 94 |
| 11 | 160 g NaOH | 37,5 g | 400 ml Methanol | 600 ml Toluol | RT | 1 h | 88 |
| 12 | 84 g NaOH | 19 g | 400 ml Methanol | 400 ml Fluor-benzol | RT | 1 h | 92 |
| 13 | 84 g NaOH | 19 g | - | 400 ml Methanol; zur Aufarbeitung Toluol | RT | 4 h | 82 |

Tabelle 2: Entsprechend Beispiel 1, Ansatz 8, wurden die in der nachstehenden Tabelle 2 aufgeführten Verbindungen hergestellt.

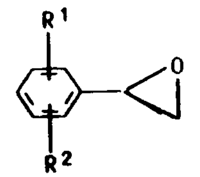

| Beispiel Nr. | $R^1$ | $R^2$ | Physik. Daten | Ausbeute (Gew.-%) (bezogen auf Halogenacetophenon) |
|---|---|---|---|---|
| 2 | p-$OCH_3$ | H | Fp. 23°C | 86 |
| 3 | p-Cl | H | $Kp_{0,8}$ 74 - 76°C | 88 |
| 4 | p-$CH_3$ | H | $Kp_{1.0}$ 51 - 53°C | 89 |
| 5 | m-$OCH_3$ | H | $Kp_{0.8}$ 76 - 79°C | 85 |
| 6 | m-$NO_2$ | H | $Kp_{0.7}$ 110 - 112°C | 92 |
| 7 | p-$NO_2$ | H | Schmp. 83°C (Petrolether) | 88 |
| 8 | p-Br | H | $Kp_{0.7}$ 72 - 74°C | 94 |

Tabelle 2: Fortsetzung - Entsprechend Beispiel 1, Ansatz 8, wurden die in der nachstehenden Tabelle 2 aufgeführten Verbindungen hergestellt.

| Beispiel Nr. | $R^1$ | $R^2$ | Physik. Daten | Ausbeute (Gew.-%) (bezogen auf Halogenacetophenon) |
|---|---|---|---|---|
| 9 | m-$NO_2$ | H | $Kp_{1.0}$ 94 - 97°C | 92 |
| 10 | 3-F | 4-F | $Kp_{0.3}$ 55 - 57°C | 86 |
| 11 | o-$NO_2$ | H | Schmp. 60 - 62°C (Ether/Petrolether) | 88 |
| 12 | 2-F | 4-F | $Kp_{0.2}$ 44 - 45°C | 79 |

## Patentansprüche

**1.** Verfahren zur Herstellung von Phenyl-substituierten Epoxiden der Formel I

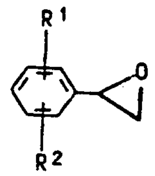

I.

worin $R^1$ und $R^2$ gleich oder verschieden sind und für Wasserstoff, $C_1$-$C_4$-Alkyl, $C_1$-$C_4$-Alkenyl, Phenyl, Halogen, Nitro, $C_1$-$C_4$-Alkoxy, $C_1$-$C_4$-Halogenalkyl, Phenoxy oder Phenylsulfonyl stehen, durch Reduktion eines Halogenacetophenons der Formel II

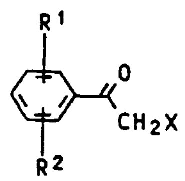

II.

worin $R^1$ und $R^2$ die oben genannten Bedeutungen besitzen und X für ein Halogenatom steht, mit einem Alkaliborhydrid zu einem Halogenhydrin der Formel III

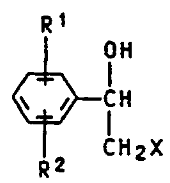

III.

worin $R^1$, $R^2$ und X die oben genannten Bedeutungen haben und Umsetzung des Halogenhyrins mit einem Alkali- oder Erdalkalihydroxid, dadurch gekennzeichnet, daß man die Lösung oder Suspension einer Mischung aus Alkaliborhydrid und Alkali- oder Erdalkalihydroxid in einem Gemisch aus Wasser und einer mit Wasser mischbaren organischen Flüssigkeit mit einer Lösung des Halogenacetophenons in einer organischen Flüssigkeit umsetzt.

**2.** Verfahren nach Anspruch 1, dadurch gekennzeichnet, daß man ein Epoxid der Formel I herstellt, in der $R^1$ Wasserstoff, Halogen oder $C_1$-$C_4$-Halogenalkyl und $R^2$ Halogen oder $C_1$-$C_4$-Halogenalkyl bedeuten.

**3.** Verfahren nach Anspruch 1, dadurch gekennzeichnet, daß man in einem Temperaturbereich von 10 ° C bis 60 ° C arbeitet.

**4.** Verfahren nach Anspruch 1, dadurch gekennzeichnet, daß man die Aufarbeitung der Reaktionsmischung durch Extraktion bei einem pH-Wert von pH = 12 - 14 oder pH = 4 - 9 vornimmt.

**5.** Verfahren nach Anspruch 1, dadurch gekennzeichnet, daß man eine Verbindung der Formel I herstellt, in der $R^1$ 4-Fluor und $R^2$ Wasserstoff bedeutet.

## Claims

**1.** A process for the preparation of a phenyl-substituted epoxide of the formula I

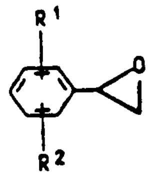

I

where $R^1$ and $R^2$ are identical or different and are each hydrogen, $C_1$-$C_4$-alkyl, $C_1$-$C_4$-alkenyl, phenyl, halogen, nitro, $C_1$-$C_4$-alkoxy, $C_1$-$C_4$-haloalkyl, phenoxy or phenylsulfonyl, by reducing a haloacetophenone of the formula II

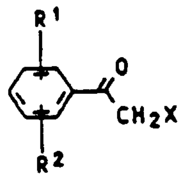

II

where $R^1$ and $R^2$ have the abovementioned meanings and X is a halogen atom, with an alkali metal borohydride to give a halohydrin of the formula III

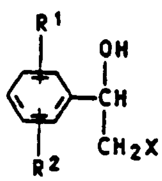

III

where $R^1$, $R^2$ and X have the abovementioned meanings, and reacting the halohydrin with an alkali metal hydroxide or alkaline earth metal hydroxide, wherein the solution or suspension of a mixture of alkali metal borohydride and alkali metal hydroxide or alkaline earth metal hydroxide in a mixture of water and a water-miscible organic liquid is reacted with a solution of haloacetophenone in an organic liquid.

2. A process as claimed in claim 1, wherein an epoxide of the formula I is prepared, in which $R^1$ is hydrogen, halogen or $C_1$-$C_4$-haloalkyl and $R^2$ is halogen or $C_1$-$C_4$-haloalkyl.

3. A process as claimed in claim 1, wherein the reaction is carried out at from 10 to 60 ° C.

4. A process as claimed in claim 1, wherein the reaction mixture is worked up by extraction at a pH of 12-14 or 4-9.

5. A process as claimed in claim 1, wherein a compound of the formula I is prepared in which $R^1$ is 4-fluoro and $R^2$ is hydrogen.

**Revendications**

1. Procédé de préparation d'époxydes substitués par phényle, de formule I

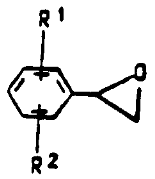

I,

dans laquelle $R^1$ et $R^2$ sont identiques ou différents et sont mis pour hydrogène, alkyle en C 1-C 4, alcényle en C 1-C 4, phényle, halogène, nitro, alcoxy en C 1-C 4, halogènalkyle en C 1-C 4, phénoxy ou phénylsulfonyle, par réduction d'une halogènacétophénone de formule II

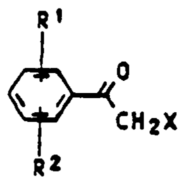

II,

dans laquelle $R^1$ et $R^2$ ont les significations données plus haut et
X est mis pour un atome d'halogène, avec un hydrure de bore alcalin, pour obtenir une halogènehydrine de formule III

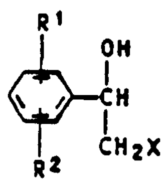

III,

dans laquelle $R^1$ et $R^2$ ont les significations données plus haut et réaction de l'halogènehydrine avec un hydroxyde alcalin ou alcalino-terreux, caractérisé par le fait que l'on met à réagir la solution ou la suspension d'un mélange d'hydrure de bore alcalin et d'hydroxyde alcalin ou alcalino-terreux dans un mélange d'eau et d'un liquide organique miscible avec l'eau, avec une solution de l'halogénacétophénone dans un liquide organique.

**2.** Procédé selon la revendication 1, caractérisé par le fait que l'on prépare un époxyde de formule I dans laquelle $R^1$ représente hydrogène, halogène ou halogènalkyle en C 1-C 4 et $R^2$, halogène ou halogènalkyle en C 1-C 4.

**3.** Procédé selon la revendication 1, caractérisé par le fait que l'on opère dans une plage de températures comprise entre 10 degrés C et 60 degrés C.

**4.** Procédé selon la revendication 1, caractérisé par le fait que l'on effectue le post-traitement du mélange réactionnel par extraction à une valeur du pH comprise entre 12 et 14 ou entre 4 et 9.

**5.** Procédé selon la revendication 1, caractérisé par le fait que l'on prépare un composé de formule I dans laquelle $R^1$ représente 4-fluor et $R^2$, hydrogène.